(19) Europäisches Patentamt
European Patent Office
Office européen des brevets

(11) Veröffentlichungsnummer: **0 283 884 B1**

(12) # EUROPÄISCHE PATENTSCHRIFT

(45) Veröffentlichungstag der Patentschrift: **12.06.91**

(51) Int. Cl.⁵: **C07C 263/10**

(21) Anmeldenummer: **88103974.7**

(22) Anmeldetag: **14.03.88**

(54) **Verfahren zur Herstellung von Diisocyanaten.**

(30) Priorität: **25.03.87 DE 3709781**

(43) Veröffentlichungstag der Anmeldung:
**28.09.88 Patentblatt 88/39**

(45) Bekanntmachung des Hinweises auf die Patenterteilung:
**12.06.91 Patentblatt 91/24**

(84) Benannte Vertragsstaaten:
**BE DE FR GB IT**

(56) Entgegenhaltungen:
**EP-A- 0 058 368**
**DE-B- 1 123 662**
**US-A- 2 963 504**
**US-A- 3 322 809**

(73) Patentinhaber: **BAYER AG**
**W-5090 Leverkusen 1 Bayerwerk(DE)**

(72) Erfinder: **Scholl, Hans Joachim, Dr.**
**Am Feldrain 5**
**W-5000 Köln 80(DE)**
Erfinder: **Klein, Alfons**
**Virchowstrasse 9**
**W-4000 Düsseldorf(DE)**

EP 0 283 884 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von gegebenenfalls als Homologen- und/oder Isomerengemische vorliegende alkylsubstituierten Diisocyanatotoluolen durch Orthoalkylierung von Diaminotoluolen zu den, den Diisocyanaten entsprechenden, am Kern alkylsubstituierten Diaminotoluolen und deren anschließende Phosgenierung.

Diisocyanatobenzole, die höhere, d.h. 8 bis 18 Kohlenstoffatome aufweisende Alkylsubstituenten tragen, stellen besonders interessante Ausgangsmaterialien für die Herstellung von Polyurethankunststoffen dar, da es sich bei diesen Diisocyanaten um Flüssigkeiten eines niedrigen Dampfdrucks handelt, die beispielsweise im Vergleich zu den homologen Diisocyanatotoluolen in physiologischer Hinsicht weit weniger problematisch sind, wobei als weiterer Vorteil hinzukommt, daß die Alkylsubstituenten nicht nur den Diisocyanaten sondern auch den aus ihnen hergestellten Zwischenprodukten (z.B. den aus den Diisocyanaten hergestellten NCO-Prepolymeren) eine verbesserte Löslichkeit in wenig polaren Lösungsmitteln verleihen. Derartige, höhere Alkylreste als substituentenaufweisende Diisocyanatobenzole bzw. ein Verfahren zu ihrer Herstellung werden beispielsweise in der DE-AS 1 123 662 (= GB-PS 852 988 = US-PS 2 986 576) beschrieben. Das dem Verfahren dieser Vorveröffentlichung zugrundeliegende alkylsubstituierte Diaminobenzol wird gemäß US-PS 2 934 571 durch Dinitrierung des entsprechenden Alkylbenzols und Hydrierung des resultierenden, alkylsubstituierten Dinitrobenzols erhalten. Nachteilhaft bei dieser Verfahrensweise ist, daß die Dinitrierung der Alkylbenzole gemäß US-PS 2 934 571 selbst unter Verwendung eines Gemischs aus rauchender Salpetersäure und rauchender Schwefelsäure als Nitriersäure nur unvollständig gelingt, so daß streng difunktionelle Diisocyanate kaum erhältlich sind. Auf diesen Sachverhalt wird in der EP-B-00 58 368 näher eingegangen. Die Autoren dieser Vorveröffentlichung machten sich zur Aufgabe die genannten Nachteile der Verfahrensweise gemäß DE-AS 1 123 662 bzw. US-PS 2 934 571 zu überwinden. Dies gelang durch Verwendung spezieller, als Homologengemische vorliegender Alkylbenzole als Ausgangsmaterial für die Nitrierung, wobei die als Ausgangsmaterialien dienenden Alkylbenzole Homologengemische darstellen, welche bei 1013 mbar einen Siedebereich von 10 bis 50 °C, vorzugsweise 20 bis 30 °C innerhalb des Temperaturbereichs von 270 bis 330 °C aufweisen und wobei die Alkylsubstituenten gesättigte, geradkettige aliphatische Kohlenwasserstoffreste mit 8 bis 15, vorzugsweise 10 bis 13 Kohlenstoffatomen darstellen. Derartige Alkylbenzol-Homologengemische müssen in einem separaten Arbeitsgang durch Alkylierung von Benzol mit den entsprechenden, linearen Olefingemischen hergestellt werden, die ihrerseits durch eine Oligomerisation von Ethylen erhalten werden. Es kann daher gesagt werden, daß es sich bei den Diisocyanaten der EP-B-00 58 368 zwar um technisch interessante Ausgangsmaterialien für den Polyurethanchemiker handelt, daß jedoch die Herstellung der Diisocyanate technisch aufwendig ist.

Es war daher die der Erfindung zugrunde liegende Aufgabe, ein Verfahren zur Herstellung von Diisocyanaten zur Verfügung zu stellen, mit dem auf technisch einfache Weise aus wohlfeilen Ausgangsmaterialien die Diisocyanate zugänglich sind.

Diese Aufgabe konnte durch das nachstehend näher beschriebene erfindungsgemäße Verfahren gelöst werden.

Bei dem nachstehend näher beschriebenen erfindungsgemäßen Verfahren werden in der 1. Stufe gegebenenfalls Isomerengemische darstellende Diaminotoluole mit einer Al/Zn-Legierung und Aluminiumchlorid bei Temperaturen von 150 - 250 °C erhitzt, nach Beendigung der Wasserstoffentwicklung mit gegebenenfalls Homologengemische darstellenden 1-Alkenen mit 8 bis 18 Kohlenstoffatomen bei Drücken von 5 bis 50 bar und Temperaturen von 250 bis 330 °C zur Reaktion gebracht und nach dem Entspannen aufgearbeitet. Diese 1. Stufe des erfindungsgemäßen Verfahrens ist für die niedrigen Alkene Ethen und Propen bereits in der DE-OS 3 402 983 beschrieben. Aus der Literatur ist ferner zu entnehmen (Angew. Chem. 69 (1957), 5. 131), daß die Reaktionsfähigkeit für diese Alkylierungsreaktion mit steigendem Molekulargewicht der Alkene abnimmt, so daß aus diesen Gründen höhere Olefine für derartige Orthoalkylierungsreaktionen bislang nicht zur Einsatz gelangten. Es muß daher als überraschend angesehen werden, daß die Umsetzung gemäß 1. Stufe des erfindungsgemäßen Verfahrens glatt in hohen Ausbeuten gelingt.

Gegenstand der Erfindung ist also ein Verfahren zur Herstellung von gegebenenfalls Isomeren- und/oder Homologengemische darstellenden Diisocyanaten, welches dadurch gekennzeichnet ist, daß man

a) gegebenenfalls Isomerengemische darstellende Diaminotoluole mit einer Al/Zn-Legierung und Aluminiumchlorid bei Temperaturen von 150 - 250 °C erhitzt, nach Beendigung der wasserstoffentwicklung mit gegebenenfalls Homologengemische darstellenden 1-Alkenen mit 8 bis 18 Kohlenstoffatomen bei Drükken von 5 bis 50 bar und Temperaturen von 250 bis 330 °C umsetzt und nach dem Entspannen das Reaktionsgemisch aufarbeitet und

b) die so erhaltenen alkylsubstituierten Diaminotoluole durch Phosgenierung in die Diisocyanate überführt.

Bei dem beim erfindungsgemäßen Verfahren zum Einsatz gelangenden Diaminotoluolen handelt es sich um 2,4-Diaminotoluol oder um dessen technischen Gemische mit bis zu 35 Gew,-%, bezogen auf Gemisch, an 2,6-Diaminotoluol. Besonders bevorzugt werden Gemische aus 2,4- und 2,6-Diaminotoluol im Gewichtsverhältnis 80:20 und 65:35 eingesetzt.

Bei dem beim erfindungsgemäßen Verfahren einzusetzenden 1-Alkenen handelt es sich um Ethylenoligomerisate mit 8 bis 18 Kohlenstoffatomen, die technisch durch Ethylenoligomerisierung erhalten werden können, und die z.B. unter der Bezeichnung "Shop-Olefin" von der Firma Shell vertrieben werden. Es kann sich bei den beim erfindungsgemäßen Verfahren zu Einsatz gelangenden Alkenen um technisch reine Verbindungen oder um Gemische von Homologen einer unterschiedlichen Kettenlänge innerhalb des genannten Bereichs von 8 bis 18 Kohlenstoffatomen handeln.

Als Al/Zn-Legierungen kommen solche mit einem Zinkgehalt von etwa 2 bis 30 Gew.-%, bevorzugt 5 bis 20 Gew.-%, infrage. Der Aluminiumgehalt beträgt entsprechend etwa 70 bis 98 Gew.-%, bevorzugt 80 bis 95 Gew.-%. Die Legierungen können in Form von Drehspänen, von Granulaten, von Grieß oder von Pulver eingesetzt werden.

Die Menge der einzusetzenden Al/Zn-Legierung kann variiert werden und hängt u.a. vom Zn-Anteil in der Al/Zn-Legierung ab. Im allgemeinen werden etwa 0,5 bis 4 Gew.-%, bevorzugt 1 bis 2 Gew.-%, Al/Zn-Legierung, bezogen auf das zu alkylierende m-Phenylendiamin, eingesetzt.

Die Menge des eingesetzten Aluminiumchlorids beträgt üblicherweise etwa 1 bis 7 Gew.-%, bevorzugt 2,5 bis 4 Gew.-%, bezogen auf das zu alkylierende Toluylendiamin.

In der Stufe a) des erfindungsgemäßen Verfahrens wird zunächst das zu orthoalkylierende Diaminotoluol mit einer Al/Zn-Legierung und dem Aluminiumchlorid (wasserfrei) so lange bei Temperaturen von etwa 150 bis 250 °C, bevorzugt 170 bis 220 °C, erhitzt bis keine Wasserstoffentwicklung mehr zu beobachten ist. Dann wird das Gemisch im Autoklaven bei Temperaturen von etwa 250 bis 330 °C, bevorzugt 280 bis 310 °C, mit den Alken umgesetzt. Die Drücke betragen dabei etwa 5 bis 50 bar, bevorzugt 10 bis 30 bar. Nach Beendigung der Alkenaufnahme wird noch etwas nachreagieren lassen und nach dem Entspannen das Reaktionsgemisch aufgearbeitet.

Die Aufarbeitung des Reaktionsgemisches erfolgt in bekannter Weise durch Versetzen des Reaktionsgemisches mit Wasser oder mit wäßriger Natronlauge, wodurch der Katalysator zersetzt wird. Die organische Phase kann anschließend einer Vakuumdestillation unterworfen werden oder nach anderen, üblichen Reinigungsverfahren aufgearbeitet werden.

In der Stufe b) des erfindungsgemäßen Verfahrens werden die so erhaltenen Diamine einer an sich bekannten Phosgenierungsreaktion unterzogen. Hierzu wird beispielsweise das Diamin in einem Hilfslösungsmittel wie Chlorbenzol gelöst und in eine Lösung von Phosgen in Chlorbenzol unter Rühren und Kühlen bei -20 bis +5 °C, vorzugsweise -10 bis 0 °C eingetropft (Kaltphosgenierung), wonach das Reaktionsgemisch unter fortlaufendem Rühren und Einleiten von Phosgen auf eine Endtemperatur von 80 bis 16 °C, vorzugsweise 100 bis 140 °C erhitzt wird, um das zunächst gebildete Carbamidsäurechlorid in das angestrebte Diisocyanat zu überführen (Heißphosgenierung). Anschließend wird das Reaktionsgemisch in an sich bekannte Weise aufgearbeitet. Selbstverständlich kann die Überführung der Diamine in die erfindungsgemäßen Diisocyanate auch nach beliebigen anderen, an sich bekannten Phosgeniermethoden erfolgen.

Die nach diesen Verfahren hergestellten Diisocyanate stellen flüssige Substanzen dar, die keine Kristallisationsneigung erkennen lassen, die, bedingt durch ihren geringen Dampfdruck, bei Raumtemperatur keinen nennenswerten Eigengeruch aufweisen, und die herstellungsbedingt keine störenden Restanteile an Monoisocyanaten aufweisen. Es handelt sich, in Abhängigkeit von der Natur der eingesetzten Produkte entweder um chemisch weitgehend einheitliche Substanzen oder um Isomeren- und/oder Homologengemische von Diisocyanaten der eingangs genannten allgemeinen Formel. Die so hergestellten Diisocyanate stellen wertvolle Ausgangsmaterialien zur Herstellung von Polyurethankunststoffen nach dem Isocyanat-Polyadditionsverfahren dar. Für diesen Verwendungszweck können die Diisocyanate anstelle der bislang eingesetzten aromatischen Diisocyanate eingesetzt werben. Dies bedeutet, daß sich die Diisocyanate hervorragend zur Herstellung von beispielsweise Polyurethanschaumstoffen, -elastomeren, -klebstoffen, dispersionen, -beschichtungen oder -lacken nach den an sich bekannten Verfahren unter Mitverwendung der an sich bekannten Reaktionspartner und Hilfsstoffe eignen. Die Diisocyanate eignen sich auch ausgezeichnet zur Herstellung von Polyurethan-Vorprodukten. Hierunter sind beispielsweise die aus der Polyurethanchemie an sich bekannten NCO-Prepolymeren oder die aus der Polyurethanchemie an sich bekannten modifizierten Polyisocyanate, insbesondere die durch teilweise Trimerisierung der Isocyanatgruppen erhältlichen, Isocyanuratgruppen aufweisenden Polyisocyanate zu verstehen. So eignen sich die Diisocyanate beispielsweise sehr gut zur Herstellung von Isocyanato-isocyanuraten, wobei die Diisocyanate als alleinige Ausgangsdiisocyanate oder im Gemisch mit anderen Diisocyanaten, beispielsweise mit 2,4-Diisocyanatotoluol einer an sich bekannten Trimerisierungsreaktion zugeführt werden. Die Mischtrimerisate der Diisocyanate mit 2,4-Diisocyanatotoluol weisen beispielsweise eine niedrigere Viskosität auf als entsprechende Mischtrimerisate von alkylsubstituierten Diisocyanaten gemäß EP-B-00 58 368 mit 2,4-Diisocyanatotoluol. Die nach dem erfindungsgemäßen Verfahren hergestellten Diisocyanate stellen außerdem wertvolle Zwischenprodukte zur Herstellung von Schädlingsbekämpfungsmitteln dar.

Die nachfolgenden Beispiele dienen der näheren Erläuterung der Erfindung. Alle Prozentangaben beziehen sich, falls nicht anderslautend erwähnt, auf Gewichtsprozente.

Beispiel

a) Orthoalkylierung von Aminotoluol.

In einem 0,7 l Autoklaven aus rostfreiem Stahl werden 200 g eines Gemischs aus 80 Gew.-Teilen 2,4-Diaminotoluol und 20 Gew.-Teilen 2,6-Diaminotoluol, 4,7 g einer Aluminium-Zink-Legierung (Gewichtsverhältnis Al:Zn = 90:10) und 8,3 g wasserfreies Aluminiumchlorid gefüllt. Das Gemisch wird dann während 1,5 h bei 200 °C gerührt, wobei sich aufgrund der Wasserstoffentwicklung ein Druck von 9 bar einstellt. Nach Abkühlen auf 140 °C wird entspannt und 275 g auf 130 °C vorgeheiztes 1-Dodecen zugesetzt. Anschließend wird während 12 h bei 300 °C gerührt. Hierbei stellt sich ein Druck von 10 bar ein. Dann wird das Reaktionsgemisch zwecks Zersetzung des Katalysators mit 150 g 10 %iger Natronlauge unter Rühren während 1 h auf Siedetemperatur erhitzt. Schließlich wird die resultierende organische Phase abgetrennt und einer fraktionierten Destillation unterzogen. Man erhält 137 g eines Dodecyl-substituierten Diaminotoluol-Isomerengemischs mit einem Siedebereich von 190 bis 205 °C/1,0 mbar. Der Vorlauf besteht aus nicht umgesetzten Ausgangsmaterialien: 173 g 1-Dodecen und 109 g Diaminotoluol.

b) Phosgenierung:

In einem 2 l Vierhalskolben, mit Rührer, Thermometer, Gaseinleitungsrohr und Rückflußkühler versehen, werden 500 g trockenes Chlorbenzol vorgelegt. Unter Rühren und Kühlung (-10 °C) werden ca. 200 g Phosgen einkondensiert, anschließend läßt man unter Kühlung bei -10 bis -50 °C 150 g Rohamingemisch gemäß Stufe a), in 150 g Chlorbenzol gelöst, zutropfen. Unter weiterem Einleiten von Phosgen erwärmt sich die Lösung anschließend ohne weitere Kühlung auf ca. 30 °C. Nach Abklingen der Wärmetönung wird langsam bis 125 °C aufgeheizt und bis zum Ende der Chlorwasserstoffentwicklung Phosgen eingeleitet (insgesamt 400 g). Das überschüssige Phosgen wird mit Stickstoff ausgeblasen und die Lösung im Vakuum eingedampft. Man erhält 175 g Rohisocyanatgemisch mit einem NCO-Gehalt von 23 % (Theorie: 24,6 %). Das so erhaltene Diisocyanat kann ohne weitere Reinigung als Ausgangsmaterial zur Herstellung von Polyurethanen verwendet werden, oder aber einer zusätzlichen destillativen Reinigung unterworfen werden: 150 g des Diisocyanatgemisches werden unter vermindertem Druck destilliert. Bei einem Druck von 1,2 mbar destillieren innerhalb des Temperaturbereichs von 188 bis 206 °C 132 g eines praktisch farblosen Gemischs von Diisocyanaten der Formel

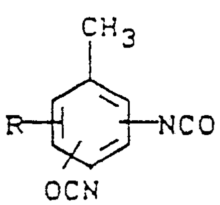

welches auch bei Abkühlung auf -50 °C keinerlei Tendenz zur Kristallbildung erkennen läßt.

**Analyse (%):**

| | NCO | C | H | N |
|---|---|---|---|---|
| gefunden: | 24,2 | 74,0 | 9,0 | 7,9 |
| Theorie: (bezogen auf $C_{21}H_{30}N_2O_2$) | 24,6 | 73,7 | 8,8 | 8,2 |

## Ansprüche

1. Verfahren zur Herstellung von, gegebenenfalls Isomeren-und/oder Homologengemische darstellenden Diisocyanaten der Formel

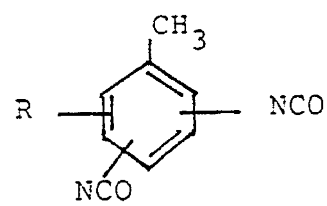

in welcher
R für einen Alkylrest mit 8 bis 18 Kohlenstoffatomen steht,
dadurch gekennzeichnet, daß man
a) gegebenenfalls Isomerengemische darstellende Diaminotoluole mit einer AL/Zn-Legierung und Aluminium-chlorid bei Temperaturen von 150 bis 250° C erhitzt, nach Beendigung der Wasserstoffentwicklung mit gegebenenfalls Homologengemische darstellenden 1-Alkenen mit 8 bis 18 Kohlenstoffatomen bei Drücken 5 bis 50 bar und Temperaturen von 250 bis 330° C umsetzt und nach dem Entspannen das Reaktionsgemisch aufarbeitet und
b) die so erhaltenen alkylsubstituierten Diaminotoluole durch Phosgenierung in die Diisocyanate überführt.

## Claims

1. A process for the preparation of diisocyanates corresponding to the following formula

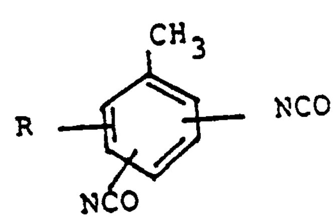

optionally as isomeric and/or homologous mixtures, in which formula,
R stands for an alkyl group having 8 to 18 carbon atoms,
characterised in that

a) diaminotoluenes, optionally consisting of isomeric mixtures, are heated to temperatures from 150 to 250 °C with an Al/Zn alloy and aluminium chloride, reacted, after termination of the evolution of hydrogen, with 1-alkenes having 8 to 18 carbon atoms, optionally consisting of homologous mixtures, at pressures from 5 to 50 bar and at temperatures from 250 to 330 °C, and worked up after release of pressure from the reaction mixture and

b) the alkyl substituted diaminotoluenes thus obtained are converted into the diisocyanates by phosgenation.

## Revendications

**1.** Procédé de préparation de diisocyanates, éventuellement à l'état de mélanges d'isomères et/ou d'homologues, de formule :

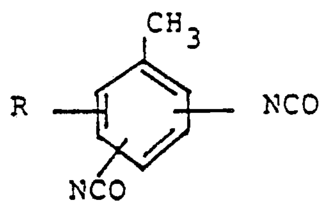

dans laquelle
R représente un groupe alkyle en $C_8$-$C_{18}$,
caractérisé en ce que

a) on chauffe des diaminotoluènes, éventuellement à l'état de mélanges d'isomères, avec un alliage Al/Zn et du chlorure d'aluminium à des températures de 150 à 250 °C; lorsque le dégagement d'hydrogène a cessé, on fait réagir avec des 1-alcènes en $C_8$-$C_{18}$ éventuellement à l'état de mélanges d'homologues, sous des pressions de 5 à 50 bar et à des températures de 250 à 330 °C et, après détente, on traite le mélange de réaction pour isolement du produit obtenu et

b) on convertit les diaminotoluènes à substituants alkyles ainsi obtenus, par phosgénation, en les diisocyanates.